(19) Europäisches Patentamt
European Patent Office
Office européen des brevets

(11) Veröffentlichungsnummer: **0 073 996**
**B1**

(12) # EUROPÄISCHE PATENTSCHRIFT

(45) Veröffentlichungstag der Patentschrift: **17.12.86**

(51) Int. Cl.⁴: **C 07 D 231/06,** D 06 L 3/12 // D01F1/10, C09K11/06

(21) Anmeldenummer: **82107678.3**

(22) Anmeldetag: **23.08.82**

(54) **Aufhellersalze und deren Verwendung für das Nassspinnen von Acrylfasern.**

(30) Priorität: **03.09.81 DE 3134942**

(43) Veröffentlichungstag der Anmeldung: **16.03.83 Patentblatt 83/11**

(45) Bekanntmachung des Hinweises auf die Patenterteilung: **17.12.86 Patentblatt 86/51**

(84) Benannte Vertragsstaaten: **CH DE FR GB LI**

(56) Entgegenhaltungen:
**AT-B-251 532**
**AT-B-291 916**
**AT-B-310 705**
**CH-A-609 977**
**DE-A-1 904 424**
**DE-A-2 434 162**
**DE-A-2 700 996**
**DE-A-2 755 023**
**DE-B-2 248 772**
**DE-B-2 534 180**

(73) Patentinhaber: **BAYER AG, Konzernverwaltung RP Patentabteilung, D-5090 Leverkusen 1 Bayerwerk (DE)**

(72) Erfinder: **Theidel, Hans, Dr., Tempelhofer Strasse 66, D-5090 Leverkusen 1 (DE)**

**EP 0 073 996 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Gegenstand der Erfindung ist die Verwendung von Pyrazolin-Aufhellern der Formel

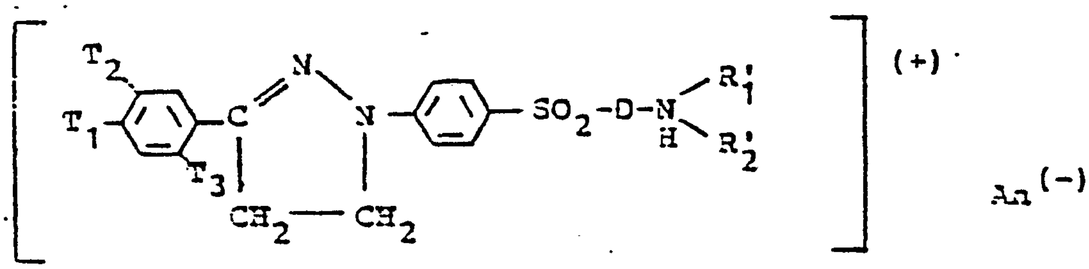

worin
$An^{(-)}$ für ein Lactat-Anion,

D für $-CH_2CH_2-$, $-CH_2CH_2CH_2-$, $-CH_2CH_2-OCH_2CH_2-$,
$-CH_2CH_2-OCH-CH-$
$CH_3$

und vorzugsweise

für $-CH_2-C-$
$CH_3$

$R_1$ und $R_2$ für $C_1$-$C_2$-Alkyl und
$T_1$, $T_2$, $T_3$ für H, $CH_3$ oder Cl stehen,
zur Einarbeitung in Spinnmassen für die Herstellung von Acrylfasern nach der Naßspinnmethode.

Die Milchsäuresalze der Formel (I) - als solche - sind bislang nicht in der Literatur beschrieben worden.

Es ist jedoch bekannt, daß man kationische Pyrazolinverbindungen - ganz allgemein - sowohl zum Aufhellen von vorgefertigten Acrylfasern nach dem Ausziehverfahren als auch nach der Naßspinnmethode ("Spinnfarben") verwenden kann. Aus der AT-A 310 705 sind weiterhin sogar Lactate von sehr ähnlichen Pyrazolinbasen bekannt, die aber aus langer Flotte eingesetzt werden (vgl. Beispiel 4).

Die vorliegenden Lactatsalze sind hervorragend geeignet zur Einarbeitung in Spinnmassen für die Herstellung von Polyacrylnitrilfsern im sogenannten Naßspinnvergahren.

In den Spinnmassen zeichnen sich die erfindungsgemäß zu verwendenden Lactate durch eine gute Langzeitlagerstabilität und vor allem durch eine absolute Beständigkeit gegenüber dem in den Spinn- und Fällungsbädern üblicherweise enthaltenden Natriumrhodanid aus.

In dieser Hinsicht sind die Lactate der Formel I auch den bislang eingesetzten Hydrochloriden, Acetaten und Quartärsalzen (beispielsweise gemäß DE-A 2 248 772) überlegen.

Die erfindungsgemäß zu verwendenden Pyrazolin-Lactate erhält man z.B. in der Weise, daß man die Lösungen der entsprechenden Pyrazolin-Basen in einem geeigneten organischen Lösungsmittel bei 50-120°C, vorzugsweise 60-80°C, mit der berechneten Menge OL-Milchsäure versetzt und das sich in fester Form abscheidende Lactat abtrennt.

Geeignete Lösungsmittel sind solche, in denen die Base gut das Salz aber wenig löslich ist, wie z.B. Dioxan, Tetrahydrofuran und vor allem Methylethylketon und Diethylketon.

Die Lactate kommen vorzugsweise in Form ihrer Flüssigiormierungen zum Einsatz. Diese erhält man wiederum durch Auflösen der festen Lactate in Wasser bei Leicht erhöhten Temperaturen (20 - 35°C). Dem Wasser werden zweckmäßigerweise geringe Mengen (bis 5 % überschuß, bezogen auf die Menge Laktat) Milchsäure, die dar Hydrolyse der Salze entgegen wirken soll, sowie gegebenenfalls hydrotrope Mittel (z.B. Harnstoff), lösungsstabilisierende Mittel (z.B. Ethylenglykoldiacatat), Konservierungsmittel, wasserlösliche

kationische Nuamciarungsfarbstoffe u.a. zugesetzt.

Vorteilhaftarweise stellt man diese Flüssigformulierungen im Eintopfverfahren her, indem man die entsprechenden Pyrazolinbasen direkt in wäßrigen Milchsäurelösungen, die die oben erwehntan Hilfsstoffe enthalten können, löst.

Die den Lactaten zugrundeliegenden Pyrazolinbasen sind bekannt und beispielsweise in folgender Patentliteratur direkt oder indirekt (als Quartärsalze) beschrieben:

DE-A 1 904 424, 2 534 180 und 2 700 996.

Die oben erwähnten Flüssigformierungen haben im allgemeinen folgende Zusammensetzung:

5 - 25, vorzugsweise 8 - 12 Gew.-% Lactat

0,1 - 1 Gew.-% Konservierungsmittel

0 - 0,5 Gew.-% Nuancierungsfeßstoff

Rest = Wasser, das zum Teil durch Milchsäure, hydrotrope Substanzen ind/oder Lösungsvermittler ersetzt sein kann.

In den damit zubereiteten Spinnbädern ist der Lactat-Wirkstoff mit 0,001 - 0,5, vorzugsweise 0,05 - 0,3 Gew.-%, enthalten.

**Beispiel 1**

In 42 ml destilliertem Wasser werden bei 40° C nacheinander 20 g Harnstoff, 30 g DL-Milchsäure (80 %ig) und 8 g pyrazolinbase der Formel

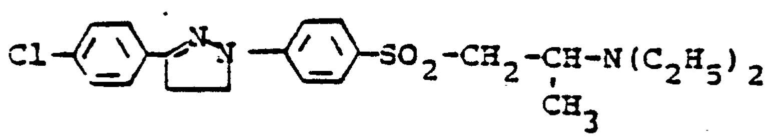

gelöst. Dann wird nach Zugabe von 0,1 g eines formaldehyiabspaltenden Konservierungsmittel durch ein Papierfilter filtriert.

Die so erhaltene Flüssigformierung wird in üblicherweise in eine rhodanidhaltige Polyacrylnitril-Spinnmasse in einer solchen Menge eingearbeitet, daß darin D,2 % Lactat-Wirkstoff enthalten sind. Nach dem Verspinnen erhält man eine einwandfrei weißgetönte Faser mit einem hohen Aufhellgrad.

**Beispiel 2**

Die Mischung aus 500 ml Methylethylketon und 70g Milchsäure (80 %ig) wird 2 Stunden unter Rückfluß zum Sieden erhitzt und nach Abkühlen auf ca. 50° C vom geringen farblosen Niederschlag abfiltriert. Dann werden bei 60° C während ca. 20 Minuten unter Rühren 100 g der Pyrazolinbase gemäß Beispiel 1 gelöst und diese Lösung bei 60-65° C ca. 2 Stunden gehalten. Dann läßt man diese unter weiterem Rühren erkalten und auskristallisieren. Der Niederschlag wird nach dem Albsaugen zweimal mit Methylethylketon/Milchsäure (80 %ig) gewaschen, bei ca. 70° C (Normaldruck) ca. 3 Stunden getrocknet, dann zerrieben ind erneut ca. 2 stunden nachgetrocknet.

Aus dem kristallreinem Lactat wird eine 11,5 %ige wäßrige, milchsaure Lösung hergestellt, der 0,2 % Konservierungsmittel zugesetzt wird.

Bei der jerwendung dieser Flüssigformierung in dem polyacrylnitril-Naßspinnprozeß erhält man ebenfalls eine einwandfrei weißgetönte Faser.

**Beispiele 3-4**

Weitere zum Naßspinnen geeignete Flüssigformierunqen erhält man, wenn man die in folgender Tabelle angegebenen Verbindungen homogen vermischt, wobei Teile = Gewichtsteile bedeuten.

Tabelle

| Beispiel | Wasser | Harnstoff | Konservierungsmittel | Milchsäure (80 %) | Pyrazolinbase |
|---|---|---|---|---|---|
| 3) | 51,6 Teile | 20 Teile | 0,1 Teile | 20 Teile | 8,3 Teile 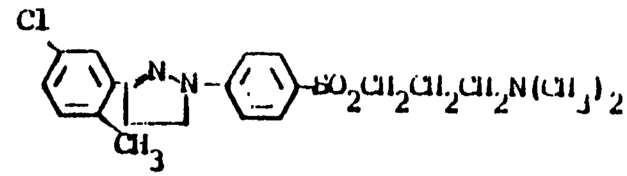 |
| 4) | 71,2 Teile | - | 0,2 Teile | 20 Teile | 8,6 Teile 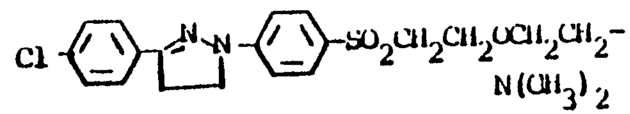 |

**Beispiel 5**

60 ml destilliertes Wasser werden bei 40°C mit 10 g DL-Milchsäure (80 %ig) vermischt, dann 8 g pyrazolinbase entsprechend Beispiel 1 gelöst sowie 0,1 g eines formaldehydabspaltenden Konservierungsmittels und 21,9 m1 EthylenglykoLdiacetat zugesetzt.

Die so erhaltene Flüssigformierung wird in üblicherweise in eine rhodanidhaltige polyacrylnitril-Spinnmasse in einer solchen Menge eingearbeitet, daß darin 0,2 % Lactat-Wirkstoff enthalten sind. Nach dem Verspinnen erhält man eine einwandfrei weißgetönte Faser mit einem hohen Aufhellgrad.

**Patentansprüche**

1. Verwendung von Pyrazolin-Aufhellern der Formel

FIG6/50

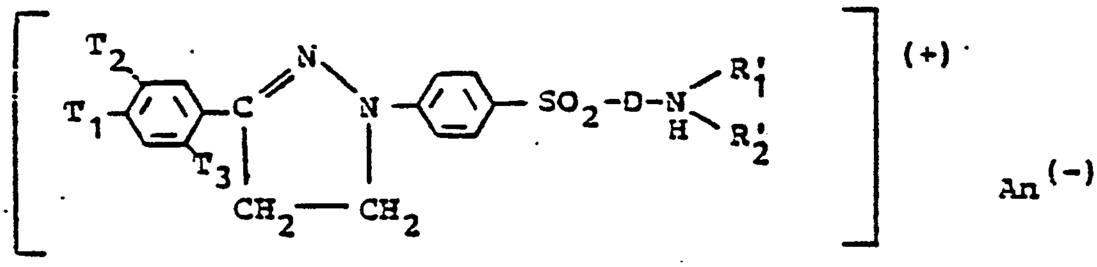

worin

An(-) für ein Lactat-Anion,

D für $-CH_2CH_2-$, $-CH_2CH_2CH_2-$, $-CH_2CH_2-OCH_2CH_2-$
$-CH_2CH_2-OCH-CH-$
$CH_3$

und vorzugsweise für

für $-CH_2-C-$
$CH_3$

$R_1$ und $R'_2$ für $C_1$-$C_2$-Alkyl und
$T_1$, $T_2$, $T_3$ für H, $CH_3$ oder Cl stehen,
zur Einarbeitung in Spinnmassen - für die Herstellung von Acrylfasern nach der Naßspinnmethode.

2. Verwendung von Pyrazolinaufhellern gemäß Anspruch 1, dadurch gekennzeichnet, daß als Aufheller eine Verbindung der Formel

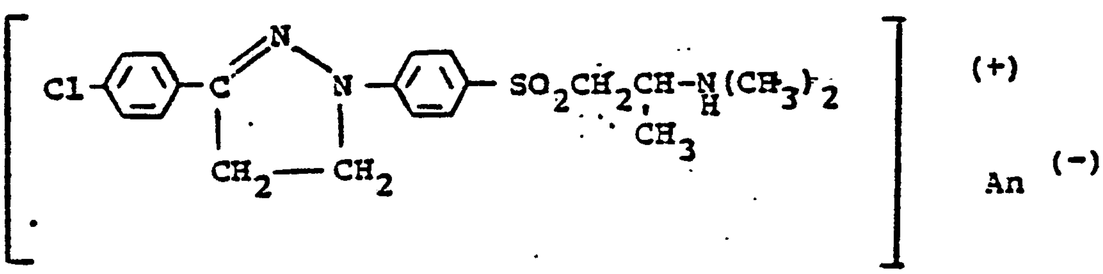

eingesetzt wird.

## Claims

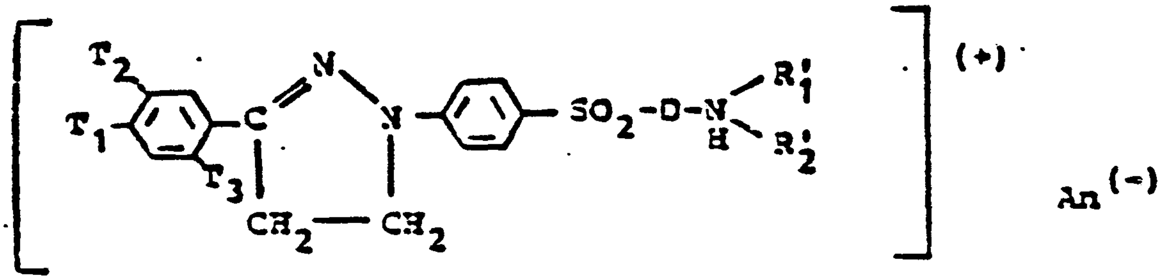

wherein
$An^{(-)}$ represents a lactate anion,

D represents $-CH_2CH_2$, $-CH_2CH_2CH_2-$, $-CH_2CH_2-OCH_2CH_2$, $-CH_2CH_2-OCH-CH-$ (with $CH_3$ on the first CH)

and preferably

$-CH_2-C-$ (with $CH_3$ on the C),

$R'_1$ and $R'_2$ represent $C_1$-$C_2$-alkyl and
$T_1$, $T_2$ and $T_3$ represent H, $CH_3$ or Cl, for incorporation in spinning masses for the production of acrylic fibres by the wet-spinning method.

2. Use of pyrazoline brighteners according to Claim 1 characterised in that a compound of the formula

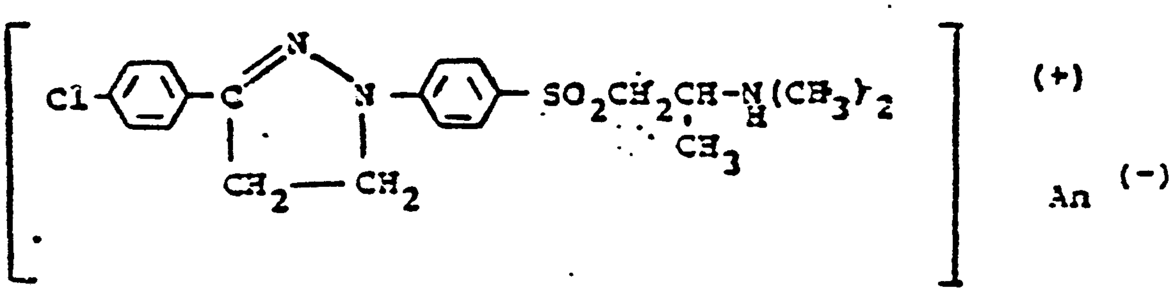

is used as the brightener.

**Revendications**

1. Utilisation d'azurants pyrazoliniques de formule

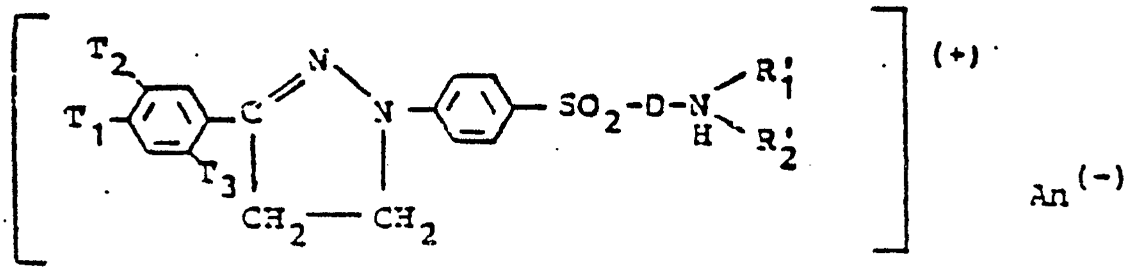

dans laquelle
$An^{(-)}$ désigne un anion lactate,

D est un groupe $-CH_2CH_2-$, $-CH_2CH_2CH_2-$,
$-CH_2CH_2-OCH_2CH_2$, $-CH_2CH_2-OCH-CH-$
$CH_3$

et de préférence un groupe

$-CH_2-C-$
$CH_3$

$R'_1$ et $R_2$ représentent un groupe alkyle en $C_1$ ou $C_2$ et
$T_1$, $T_2$, $T_3$ représentent H, $CH_3$ ou Cl, en vue de leur incorporation dans des mélanges à filer pour la production de fibres acryliques par le procédé de filage au mouillé.

2. Utilisation d'azurants pyrazoliniques suivant la revendication 1, caractérisée en ce que l'azurant utilisé est un composé de formule